(19) Europäisches Patentamt
European Patent Office
Office européen des brevets

(11) Veröffentlichungsnummer : **0 170 069**
**B1**

# (12) EUROPÄISCHE PATENTSCHRIFT

(45) Veröffentlichungstag der Patentschrift : **11.10.89**

(21) Anmeldenummer : **85108107.5**

(22) Anmeldetag : **29.06.85**

(51) Int. Cl.[4] : **A 61 K 7/13**, C 07 C101/52, C 07 C143/58, C 07 D295/08, C 07 D295/14

## (54) Haarfärbemittel.

(30) Priorität : **07.07.84 DE 3425151**

(43) Veröffentlichungstag der Anmeldung : **05.02.86 Patentblatt 86/06**

(45) Bekanntmachung des Hinweises auf die Patenterteilung : **11.10.89 Patentblatt 89/41**

(84) Benannte Vertragsstaaten : **AT BE CH DE FR GB IT LI NL SE**

(56) Entgegenhaltungen :
**DE–C– 204 884**
**DE–C– 418 989**
**US–A– 4 066 457**
**US–A– 4 189 589**
**JOURNAL OF THE CHEMICAL SOCIETY, Perkin Transactions I, Band 21, 1974, Seiten 2451-2458; J. MARTIN et al.: "Heterocyclic syntheses. Part XXVIII. ortho-dialkylamino-benzene-sulphonyl azides, - benzoyl azides, and -benzoyldiazomethanes"**
**CHEMICAL ABSTRACTS, Band 59, 1963, Nr. 12807-12809, Columbus, Ohio, US; P. CLARKE et al.: "Synthesis of some 6-substituted 1,2,3,4-tetrahydroquinoxalines"**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Rose, David, Dr.**
**Holbeinweg 7**
**D-4010 Hilden (DE)**
Erfinder : **Lieske, Edgar**
**Hunsrückstrasse 40**
**D-4000 Düsseldorf (DE)**
Erfinder : **Maak, Norbert, Dr.**
**Im Jagdfeld 41a**
**D-4040 Neuss (DE)**

**EP 0 170 069 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist die Verwendung von Nitro-aminobenzoesäuren und Nitroaminobenzolsulfonsäuren als direktziehende Haarfarbstoffe in Haarfärbemitteln. Solche Haarfärbemittel enthalten direktziehende Haarfarbstoffe in einem kosmetischen Träger. Oft enthalten solche Haarfärbemittel zur Erzielung bestimmter Nuancen zusätzlich oxidationsfarbstoffvorprodukte. Als kosmetische Träger für die direktziehenden Haarfarbstoffe und ggf. Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Unter den direktziehenden Nitrobenzolderivaten spielen dir Nitroaniline und deren Derivate eine besondere Rolle, da einige dieser Farbstoffe intensive Färbungen von guter Lichtechtheit ausbilden. Ein Nachteil der bekannten direktziehenden Nitroanilin-Farbstoffe ist jedoch die unbefriedigende Waschechtheit, d. h. daß die Haarfärbungen nach mehrmaligem Haarwaschen stark ausbluten. Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Einige Nitro-aminobenzoesäuren und Nitro-aminobenzolsulfonsäuren sind als Vorstufen zur Herstellung von Diazoverbindungen aus DE 418 989 C, als Vorstufen zur Herstellung optischer Aufheller aus US 4 189 589 A, als Vorstufen zur Herstellung von Benzoyl-Aziden und Sulfonyl-Aziden aus Journ. Chem. Soc. Perkin Trans. I, 21 (1974), S. 2451-2458, als Vorstufe zur Herstellung von Entwicklern für die Farbphotographie aus US 4 066 457 A, als Vorstufe zur Herstellung von 6-substituierten 1,2,3,4-Tetrahydrochinoxalinen aus Chem. Abstr. 59 (1963), Nr. 12807-12809 und als Vorprodukte zur Synthese von Farbstoffen aus DE 204 884 C bekannt.

Es wurde gefunden, daß die Verwendung von Verbindungen der allgemeinen Formel I

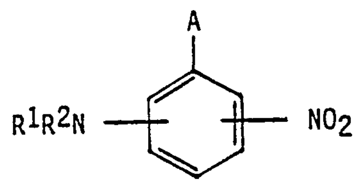

in welcher A eine $SO_3H$-Gruppe oder eine COOH-Gruppe, $R^1$ und $R^2$ Wasserstoff, eine Alkylgruppe mit 1-4 C-Atomen, eine Gruppe $-(CH_2)_n-x$, in der $n = 2$-$4$ ist und x eine Hydroxylgruppe oder eine Gruppe $-NR^3R^4$ darstellt, in der $R^3$ und $R^4$ Wasserstoff, eine Alkylgruppe mit 1-4 C-Atomen, eine Hydroxyalkylgruppe mit 2-4 C-Atomen oder eine Aminoalkylgruppe mit 2-4 C-Atomen darstellt oder $R^1$ und $R^2$ gemeinsam mit dem Stickstoffatom einen Piperidin-, Pyrrolidin-, Piperazin- oder Morpholinring bilden oder deren wasserlöslichen Salzen als direktziehende Haarfarbstoffe in Haarfärbemitteln, die gestellten Anforderungen in hohem Maße erfüllt. Die Nitro- und die Aminogruppe können in den Verbindungen der allgemeinen Formel I in beliebiger Position zur Gruppe A und zueinander angeordnet sein. Die Farbstoffe der allgemeinen Formel I erzeugen auf dem Haar gelbe bis olivbraune Farbnuancen von hoher Intensität, Licht- und Waschechtheit. Sie besitzen im Vergleich zu bekannten Nitroanilin-Farbstoffen eine bessere Löslichkeit in wäßrig-alkalischem Medium. In dermatologischer und toxikologischer Hinsicht sind die Verbindungen unschädlich.

Die Herstellung der Verbindungen der allgemeinen Formel I erfolgt nach literaturbekannten Verfahren. Sie werden im allgemeinen durch Umsetzung der entsprechenden Chlor-nitro-benzol-sulfonsäure bzw. der entsprechenden Chlor-nitro-benzoesäure mit primären oder sekundären Aminen gemäß dem allgemeinen Reaktionsschema

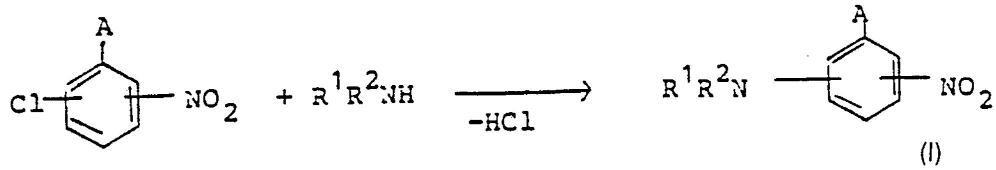

(I)

hergestellt. Dabei haben die Substituenten A, $R^1$ und $R^2$ die für die Formel I angegebene Bedeutung. Die Herstellung einiger literaturunbekannter Verbindungen der Formel I wird in Beispiel 1-6 beschrieben.

Geeignete Verbindungen der allgemeinen Formel I, in der A eine $SO_3H$-Gruppe darstellt, sind z. B.

2-Amino-3-nitrobenzolsulfonsäure
3-Amino-2-nitrobenzolsulfonsäure
4-Amino-2-nitrobenzolsulfonsäure
2-Amino-4-nitrobenzolsulfonsäure
4-Piperidino-3-nitrobenzolsulfonsäure
4-(β-Hydroxyethylamino)-3-nitrobenzolsulfonsäure
4-Methylamino-3-nitrobenzolsulfonsäure
2-Piperidino-5-nitrobenzolsulfonsäure
2-Morpholino-5-nitrobenzolsulfonsäure
2-Pyrrolidino-5-nitrobenzolsulfonsäure
2-(β-Hydroxyethylamino)-5-nitrobenzolsulfonsäure
2-Bis (β-hydroxyethyl)-amino-5-nitrobenzolsulfonsäure
2-Ethylamino-5-nitrobenzolsulfonsäure
2-Dimethylamino-5-nitrobenzolsulfonsäure

Geeignete Verbindungen der Formel I, in der A eine COOH-Gruppe darstellt, sind z. B.

2-Amino-4-nitrobenzoesäure
2-Methylamino-5-nitrobenzoesäure
2-(β-Hydroxyethylamino)-5-nitrobenzoesäure
2-Dimethylamino-5-nitrobenzoesäure
2-Piperidino-5-nitrobenzoesäure
2-Morpholino-5-nitrobenzoesäure
4-Amino-3-nitrobenzoesäure
4-Diethylamino-3-nitrobenzoesäure
4-Methylamino-3-nitrobenzoesäure
4-Ethylamino-3-nitrobenzoesäure
4-Dimethylamino-3-nitrobenzoesäure
4-Morpholino-3-nitrobenzoesäure
4-Piperidino-3-nitrobenzoesäure
4-Pyrrolidino-3-nitrobenzoesäure
4-(β-Hydroxyethylamino)-3-nitrobenzoesäure
3-Amino-2-nitrobenzoesäure
2-Amino-3-nitrobenzoesäure
4-(2-Dimethylamino)-ethylamino-3-nitrobenzoesäure
5-Amino-2-nitrobenzoesäure

Unter den Verbindungen der Formel I, in welchen A eine $SO_3H$-Gruppe ist, stellen 2-Piperidino-5-nitrobenzolsulfonsäure und 4-(β-Hydroxyethyl)-amino-3-nitrobenzolsulfonsäure besonders bevorzugte direktziehende Haarfarbstoffe dar. Unter den Verbindungen der Formel I, in welchen A eine COOH-Gruppe ist, stellen 4-Methylamino-3-nitrobenzoesäure, 4-Ethylamino-3-nitrobenzoesäure, die 2-Piperidino-5-nitrobenzoesäure, 5-Amino-2-nitrobenzoesäure und 2-Amino-4-nitrobenzoesäure besonders gut geeignete direktziehende Haarfarbstoffe dar.

Die Haarfärbemittel können die direktziehenden Nitroanilinderivate der allgemeinen Formel I allein oder in Kombination mit bekannten direktziehenden Farbstoffen, z. B. mit anderen Nitrobenzolderivaten, Anthrachinonfarbstoffen oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2.4.5.6-Tetraaminopyrimidin und dessen Derivate eingesetzt. Als Kupplersubstanzen werden

m-Phenylendiaminderivate, Naphthole, Resorcin und Resorcinderivate, Pyrazolone und m-Aminophenole verwendet.

Zur Herstellung der Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride Fettsäurealkanolamide sowie Verdickungsmittel wie z. B.

Methyl- oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt ; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den Haarfärbemitteln werden die direktziehenden Haarfarbstoffe der allgemeinen Formel I in einer Menge von 0,01 bis 5,0 Gew.-%, bevorzugt von 0,1-2 Gew.-%, bezogen auf das gesamte Haarfärbemittel, eingesetzt. Daneben können bekannte Oxidationshaarfärbemittelvorprodukte (Entwickler- und Kupplersubstanzen) in einer Menge von 0,01-5, vorzugsweise von 1-3 Gew.-% enthalten sein.

Wenn das Haarfärbemittel Oxidationsfarbstoffvorprodukte enthält, empfiehlt sich außerdem die Zugabe einer kleinen Menge eines Reduktionsmittels, z. B. von 0,6-2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die erfindungsgemäße Verwendung der Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8-10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern ohne ihn jedoch hierauf zu beschränken.

## Beispiele

1. 4-(β-Hydroxyethyl) amino-3-nitrobenzolsulfonsäure-Na-Salz

Eine Mischung, bestehend aus 20 g 4-Chlor-3-nitrobenzolsulfonsäure-Na-Salz (0,077 Mol) und 9,4 g Ethanolamin (0,154 Mol) in 100 ml Wasser wurde 6 Stunden auf 120 °C erhitzt.

Nach dem Einengen der Lösung durch Destillation unter vermindertem Druck wurde der Rückstand aus Ethanol umkristallisiert. Es wurde ein gelbes Pulver mit einem Schmelzpunkt von 307 °C erhalten.

2. 2-(β-Hydroxyethyl)-amino-5-nitrobenzolsulfonsäure-Na-Salz

Eine Mischung, bestehend aus 20 g 2-Chlor-5-nitrobenzolsulfonsäure-Na-Salz (0,077 Mol) und 9,4 g Ethanolamin (0,154 Mol) in 100 ml Wasser wurde 6 Stunden auf 120 °C erhitzt.

Nach dem Einengen der Lösung durch Destillation unter vermindertem Druck wurde der Rückstand aus Ethanol umkristallisiert. Es wurde ein gelbes Pulver mit einem Schmelzpunkt von 254 °C erhalten.

3. 2-Bis (β-hydroxyethyl) amino-5-nitrobenzolsulfonsäure-Na-Salz

Die Herstellung erfolgte analog Beispiel 2, aber unter Verwendung von 20 g Diethanolamin. Es wurde ein gelbes Pulver erhalten, dessen Schmelzpunkt oberhalb 305 °C liegt.

4. 2-Ethylamino-5-nitrobenzolsulfonsäure-Na-Salz

Die Herstellung erfolgte analog Beispiel 2, aber unter Verwendung von 30 g Diethylamin. Es wurde ein gelbes Pulver erhalten, dessen Schmelzpunkt bei 245 °C liegt.

5. 2-(β-Hydroxyethylamino)-5-nitrobenzoesäure

Eine Mischung, bestehend aus 10,1 g 2-Chlor-5-nitrobenzoesäure (0,05 Mol), 6 ml Ethanolamin (0,1 Mol) und 50 ml Wasser wurde 7 Stunden auf 170 °C erhitzt.

Nach dem Abkühlen auf + 10 °C wurde der Niederschlag abfiltriert und aus Ethanol umkristallisiert. Es wurde ein gelbes Pulver mit einem Schmelzpunkt von 139 °C erhalten.

6. 4-(2-Dimethylaminoethyl)-amino-3-nitrobenzoesäure

Die Herstellung erfolgte analog Beispiel 5, ausgehend von 10,1 g 4-Chlor-3-nitrobenzoesäure (0,05 Mol) und 20 g N.N-Dimethylethylendiamin (0,15 Mol). Es wurde ein gelbes Pulver mit einem Schmelzpunkt von 281 °C (unter Zersetzung) erhalten.

7. Haarfärbeversuche

Es wurden Haarfärbecremes der folgenden Zusammensetzung hergestellt:

| | |
|---|---|
| Fettalkohol $C_{12-18}$ | 10 g |
| Fettalkohol $C_{12-14}$ + 2 EO-sulfat, Na-Salz (28 %ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschliessend getrocknet.

Als direktziehende Haarfarbstoffe wurden die folgenden Verbindungen eingesetzt:

7.1 2-Piperidino-5-nitrobenzolsulfonsäure
7.2 4-(β-Hydroxyethyl)-amino-3-nitrobenzolsulfonsäure, Na-Salz
7.3 4-Dimethylamino-3-nitrobenzolsulfonsäure, Na-Salz
7.4 4-Piperidino-3-nitrobenzolsulfonsäure, Na-Salz
7.5 2-Morpholino-5-nitrobenzolsulfonsäure, Na-Salz
7.6 2-Dimethylamino-5-nitrobenzolsulfonsäure, Na-Salz
7.7 2-Ethylamino-5-nitrobenzolsulfonsäure, Na-Salz
7.8 2-Bis-(β-hydroxyethyl)-amino-5-nitrobenzolsulfonsäure, Na-Salz
7.9 2-(β-Hydroxyethyl)-amino-5-nitrobenzolsulfonsäure, Na-Salz
7.10 2-Pyrrolidino-5-nitrobenzolsulfonsäure, Na-Salz
7.11 2-Amino-5-nitrobenzolsulfonsäure
7.12 4-Amino-2-nitrobenzolsulfonsäure
7.13 2-Amino-4-nitrobenzoesäure
7.14 4-Methylamino-3-nitrobenzoesäure
7.15 4-Ethylamino-3-nitrobenzoesäure
7.16 2-(β-Hydroxyethyl)-amino-5-nitrobenzoesäure
7.17 2-Dimethylamino-5-nitrobenzoesäure
7.18 2-Piperidino-5-nitrobenzoesäure
7.19 4-Dimethylamino-3-nitrobenzoesäure
7.20 2-Methylamino-5-nitrobenzoesäure
7.21 2-Morpholino-5-nitrobenzoesäure
7.22 2-Pyrrolidino-5-nitrobenzoesäure
7.23 4-(2-Dimethylaminoethyl)-amino-3-nitrobenzoesäure
7.24 2-Dimethylamino-5-nitrobenzoesäure
7.25 4-Pyrrolidino-3-nitrobenzoesäure
7.26 4-Morpholino-3-nitrobenzoesäure
7.27 4-Piperidino-3-nitrobenzoesäure
7.28 4-Dimethylamino-3-nitrobenzoesäure

Das Ergebnis der Färbeversuche ist der Tabelle I zu entnehmen:

Tabelle I

| direktziehender Farbstoff | Nuance |
|---|---|
| 7.1 | olivgelb |
| 7.2 | olivbraun |
| 7.3 | honiggelb |
| 7.4 | lehmfarbig |

| | |
|---|---|
| 7.5 | oliv |
| 7.6 | olivgelb |
| 7.7 | olivgelb |
| 7.8 | khaki |
| 7.9 | oliv |
| 7.10 | olivgelb |
| 7.11 | hellgelb |
| 7.12 | beige |
| 7.13 | gelb |
| 7.14 | olivbraun |
| 7.15 | honiggelb |
| 7.16 | olivgelb |
| 7.17 | khaki |
| 7.18 | khaki |
| 7.19 | lehmfarbig |
| 7.20 | olivgelb |
| 7.21 | khaki |
| 7.22 | oliv |
| 7.23 | khaki |
| 7.24 | olivgelb |
| 7.25 | olivbraun |
| 7.26 | graugelb |
| 7.27 | dunkelblond |
| 7.28 | olivbraun |

## Patentansprüche

1. Verwendung von Verbindungen der allgemeinen Formel

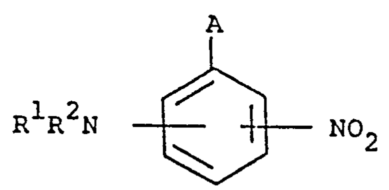

(I)

in welchen A eine $SO_3H$-Gruppe oder eine COOH-Gruppe, $R^1$ und $R^2$ Wasserstoff, eine Alkylgruppe mit 1-4 C-Atomen, eine Gruppe $—(CH_2—)_n$ X, in der n = 2-4 ist und X eine Hydroxylgruppe oder eine Gruppe $—NR^2R^4$ darstellt, in der $R^3$ und $R^4$ Wasserstoff, eine Alkylgruppe mit 1-4 C-Atomen, eine Hydroxyalkylgruppe mit 2-4 C-Atomen oder eine Aminoalkylgruppe mit 2-4 C-Atomen darstellt oder $R^1$ und $R^2$ gemeinsam mit dem Stickstoffatom einen Piperidin-, Pyrrolidin-, Piperazin- oder Morpholinring bilden oder deren wasserlöslichen Salzen als direktziehende Haarfarbstoffe in Haarfärbemitteln.

2. Verwendung nach Anspruch 1, dadurch gekennzeichnet, daß als direktziehender Haarfarbstoff wenigstens eine Verbindung aus der Gruppe

2-Piperidino-5-nitrobenzolsulfonsäure
4-(β-Hydroxyethyl)-amino-3-nitro-benzolsulfonsäure

oder deren wasserlösliches Salz eingesetzt wird.

3. Verwendung nach Anspruch 1, dadurch gekennzeichnet, daß als direktziehender Haarfarbstoff wenigstens eine Verbindung aus der Gruppe

4-Methylamino-3-nitrobenzoesäure
4-Ethylamino-3-nitrobenzoesäure
2-Piperidino-5-nitro-benzoesäure
2-Amino-4-nitrobenzoesäure

oder deren wasserlösliches Salz eingesetzt wird.

4. Verwendung nach Anspruch 1-3, dadurch gekennzeichnet, daß die direktziehenden Haarfarbstoffe in einer Menge von 0,01 bis 5 Gew.-%, bezogen auf das gesamte Haarfärbemittel eingesetzt werden.

5. Verwendung nach Anspruch 1-4, dadurch gekennzeichnet, daß zusätzlich bekannte Oxidationshaarfärbemittelvorprodukte in einer Menge von 0,01-5, vorzugsweise von 1-3 Gew.-%, eingesetzt werden.

## Claims

1. The use of compounds corresponding to the following general formula

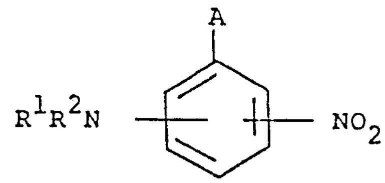

(I)

in which A is an $SO_3H$ group or a COOH group, $R^1$ and $R^2$ represent hydrogen, a $C_1$-$C_4$ alkyl group, a group $—(CH_2—)_nX$, where n = 2-4 and X is a hydroxyl group, or a group $—NR^2R^4$, where $R^3$ and $R^4$ represent hydrogen, a $C_1$-$C_4$ alkyl group, a $C_2$-$C_4$ hydroxyalkyl group or a $C_2$-$C_4$ aminoalkyl group, or $R^1$ and $R^2$ together with the nitrogen atom form a piperidine, pyrrolidine, piperazine or morpholine ring, or water-soluble salts thereof as substantive dyes in hair coloring preparations.

2. The use claimed in claim 1, characterized in that at least one compound from the group comprising

2-piperidino-5-nitrobenzene sulfonic acid and
4-(β-hydroxyethyl)-amino-3-nitrobenzene sulfonic acid

or a water soluble salt thereof is used as the substantive dye.

3. The use claimed in claim 1, characterized in that at least one compound from the group comprising

4-methylamino-3-nitrobenzoic acid,
4-ethylamino-3-nitrobenzoic acid,
2-piperidino-5-nitrobenzoic acid and
2-amino-4-nitrobenzoic acid

or a water-soluble salt thereof is used as the substantive dye.

4. The use claimed in claims 1 to 3, characterized in that the substantive dyes are used in a quantity of from 0.01 to 5 % by weight, based on the hair coloring preparation as a whole.

5. The use claimed in claims 1 to 4, characterized in that known oxidation hair dye precursors are additionally used in a quantity of from 0.01 to 5 % by weight and preferably in a quantity of from 1 to 3 % by weight.

## Revendications

1. Utilisation de composés de formule générale I

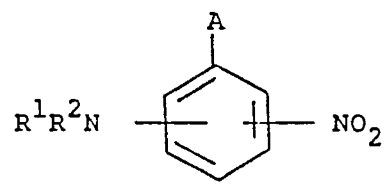

(I)

dans laquelle A représente un groupe $SO_3H$ ou un groupe COOH, $R^1$ et $R^2$ représentent un atome d'hydrogène ou un groupe alkyle ayant de 1 à 4 atomes de carbone, un groupe $—(CH_2)_n—x$, dans lequel n vaut de 2 à 4 et x représente un groupe hydroxyle ou un groupe $—NR^3R^4$, dans lequel $R^3$ et $R^4$ représentent un atome d'hydrogène ou un groupe alkyle ayant de 1 à 4 atomes de carbone, un groupe hydroxyalkyle ayant de 2 à 4 atomes de carbone ou un groupe aminoalkyle ayant de 2 à 4 atomes de carbone, ou $R^1$ et $R^2$ forment ensemble avec l'atome d'azote un cycle pipéridine, pyrrolidine, pipérazine ou morpholine, ou de leurs sels solubles dans l'eau, en tant que colorants capillaires directs, dans des produits de teinture pour cheveux.

2. Utilisation selon la revendication 1, caractérisée en ce que l'on utilise, en tant que colorant capillaire direct, au moins un composé choisi parmi

l'acide 2-pipéridino-5-nitrobenzènesulfonique,
l'acide 4-(β-hydroxyéthyl)-amino-3-nitrobenzènesulfonique

ou un sel soluble dans l'eau de ceux-ci.

3. Utilisation selon la revendication 1, caractérisée en ce que l'on utilise, en tant que colorant capillaire direct, au moins un composé choisi parmi l'acide

l'acide 4-méthylamino-3-nitrobenzoïque
l'acide 4-éthylamino-3-nitrobenzoïque
l'acide 2-pipéridino-5-nitrobenzoïque

l'acide 2-amino-4-nitrobenzoïque

ou un sel soluble dans l'eau de ceux-ci.

4. Utilisation selon l'une quelconque des revendications 1 à 3, caractérisée en ce que l'on utilise des colorants capillaires directs en une quantité de 0,01 à 5 % en poids, par rapport au produit de teinture pour cheveux total.

5. Utilisation selon l'une quelconque des revendications 1 à 4, caractérisée en ce que l'on utilise en outre des précurseurs connus de colorants capillaires d'oxydation, en une quantité de 0,01 à 5, de préférence de 1 à 3 % en poids.